# EUROPEAN PATENT APPLICATION

(11) **EP 4 782 544 A1**
(43) Date of publication of application: **29.07.2026**
(21) Application number: 24908168.8
(22) Date of filing: 20.12.2024
(51) Int. Cl.: C12N 15/77, C07K 14/34, C12P 13/08

(54) **MICROORGANISM WITH ENHANCED ACTIVITY OF AROMATIC AMINO ACID TRANSPORTER AND USES THEREOF**

(30) Priority: 22.12.2023 KR 20230190244
(71) Applicant: CJ Cheiljedang Corporation, Seoul 04560 (KR)
(72) Inventor: YUN, Hyojin, Seoul 04560 (KR); LEE, Heeseok, Seoul 04560 (KR); LEE, Hyein, Seoul 04560 (KR); KIM, Seon Hye, Seoul 04560 (KR)
(74) Representative: Meissner Bolte Partnerschaft mbB
(86) International application number: PCT/KR2024/020841
(87) International publication number: WO 2025/135886

(57) **Abstract**

The present disclosure provides a gene expression cassette, a microorganism with enhanced activity of an aromatic amino acid transporter, a method for producing valine using the microorganism, and a method for increasing valine production.

## Description

### [Technical Field]

### CROSS-REFERENCE TO RELATED PATENT APPLICATION(S)

The present disclosure claims the benefit of Korean Patent Application No. 10-2023-0190244, filed on December 22, 2023, the disclosure of which is incorporated herein by reference in its entirety.

The present disclosure relates to a microorganism having enhanced activity of an aromatic amino acid transporter and a use thereof.

### [Background Art]

Production of branched-chain amino acids using microorganisms is mainly accomplished by microorganisms of the genus *Escherichia* or microorganisms of the genus *Corynebacterium*, and in the case of L-valine, it is known that 2-oxoisovalerate is biosynthesized as a precursor from pyruvic acid through several steps, but L-valine production through microorganisms is not easy to produce in large quantities industrially.

Therefore, research is still needed to effectively increase L-valine production capability.

### [Prior Art Literature]

### [Patent Literature]

(Patent Literature 1) U.S. Patent Publication No. US 2020-0362374 A1

### [Disclosure]

### [Technical Problem]

An object of the present disclosure is to provide a microorganism having enhanced activity of an aromatic amino acid transporter and increased valine production capability.

Another object of the present disclosure is to provide a gene expression cassette comprising a promoter and a gene encoding an aromatic amino acid transporter operably linked to the promoter.

Still another object of the present disclosure is to provide a method for producing valine, comprising culturing the microorganism in a medium.

Still another object of the present disclosure is to provide a method for increasing valine production, comprising culturing the microorganism in a medium.

Yet another object of the present disclosure is to provide a composition for producing valine, comprising one or more selected from the group consisting of the microorganism and a medium in which the microorganism has been cultured.

### [Technical Solution]

A detailed description thereof is as follows. Meanwhile, each of the descriptions and embodiment disclosed in the present disclosure may also be applied to other descriptions and embodiments. That is, all combinations of various elements disclosed in the present disclosure fall within the scope of the present disclosure. In addition, the scope of the present disclosure may not be considered limited by the specific description described below. In addition, numerous papers and patent literatures are referenced and cited throughout this specification. The disclosures of the cited papers and patent literatures are incorporated herein by reference in their entirety to more clearly explain the level of the technical field to which the present invention pertains and the contents of the invention.

In the present disclosure, it was confirmed that enhancement of the activity of an aromatic amino acid transporter (aroP) resulted in an increase in valine production capability and yield as well as a decrease in production of by-products other than valine. Therefore, the present disclosure provides a promoter for enhancing the activity of an aromatic amino acid transporter and a microorganism producing valine, having enhanced activity of an aromatic amino acid transporter.

In the present disclosure, the aromatic amino acid transporter (or aroP protein) may refer to a protein having an aromatic amino acid transport activity. The aromatic amino acid transporter may be derived from a microorganism of the genus *Corynebacterium*. The aromatic amino acid transporter may be derived from *Corynebacterium glutamicum*. A sequence of the aromatic amino acid transporter may be obtained from a known database (NCBI) or the like (Sequence ID: WP_003863791.1), and may comprise, for example, the amino acid sequence of SEQ ID NO: 1 or consist of the amino acid sequence of SEQ ID NO: 1. In one embodiment, the aromatic amino acid transporter may comprise the amino acid sequence of SEQ ID NO: 1 or have 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more homology or identity with the amino acid sequence of SEQ ID NO: 1, comprise the amino acid sequence, or consist of the amino acid sequence, but is not limited thereto.

In addition, when the amino acid sequence exhibits an efficacy corresponding to the aromatic amino acid transporter in terms of homology or identity, a variant having an amino acid sequence in which some of the sequence is deleted, modified, substituted, conservatively substituted, or added may also be included in the aromatic amino acid transporter. For example, it is a case of having addition or deletion of a sequence that does not alter the activity of the aromatic amino acid transporter at the N-terminus, the C-terminus, and/or an internal region of the amino acid sequence, a naturally occurring mutation, a silent mutation, or a conservative substitution.

The "conservative substitution" refers to substituting one amino acid with another amino acid having similar structural and/or chemical characteristics. Such an amino acid substitution may generally occur based on similarities in polarity, charge, solubility, hydrophobicity, hydrophilicity, and/or amphipathic nature of residues. Generally, the conservative substitution may have almost no effect or no effect on the activity of a protein or a polypeptide.

The aromatic amino acid transporter may be a protein encoded by an aromatic amino acid transporter gene and having aromatic amino acid transporter activity. The aromatic amino acid transporter gene may be derived from a microorganism of the genus *Corynebacterium*. The aromatic amino acid transporter gene may be derived from *Corynebacterium glutamicum*. The sequence of the aromatic amino acid transporter gene may be obtained from a known database (NCBI) or the like. The aromatic amino acid transporter gene may comprise the nucleic acid sequence of SEQ ID NO: 2 or may consist of the nucleic acid sequence of SEQ ID NO: 2. In one embodiment, the aromatic amino acid transporter gene may have 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more homology or identity with the nucleic acid sequence of SEQ ID NO: 2, comprise the nucleic acid sequence, or consist of the nucleic acid sequence, but is not limited thereto.

In the present disclosure, the expression that a polynucleotide or polypeptide "has, comprises a specific nucleic acid sequence (nucleotide sequence) or amino acid sequence, consists of the sequence, or essentially consists of the sequence" may mean that the polynucleotide or polypeptide essentially comprises the specific nucleic acid sequence or amino acid sequence, and may be interpreted to include a "substantially equivalent sequence" in which a mutation (deletion, substitution, modification, and/or addition) is applied to the specific nucleic acid sequence or amino acid sequence within the scope of maintaining the original function and/or desired function of the polynucleotide or polypeptide (or not to exclude the mutation). In one embodiment, the expression that a polynucleotide or polypeptide "has, comprises a specific nucleic acid sequence (nucleotide sequence) or amino acid sequence, consists of the sequence, or essentially consists of the sequence" may mean that the polynucleotide or polypeptide (i) comprises the specific nucleic acid sequence or amino acid sequence, or (ii) consists of or comprises a nucleic acid sequence or amino acid sequence having 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more homology or identity with the specific nucleic acid sequence or amino acid sequence, while maintaining the original function and/or desired function.

In the present specification, the term "homology" or "identity" refers to a degree of similarity between two given amino acid sequences or nucleotide sequences and may be expressed as a percentage. The terms "homology" and "identity" may often be used interchangeably.

Sequence homology or identity of a conserved polynucleotide or polypeptide may be determined by a standard alignment algorithm, and a default gap penalty established by a program being used may be used together. Substantially, homologous or identical sequences may generally hybridize with all or part of the sequence under moderate or high stringency conditions. It is obvious that hybridization also comprises hybridization with a polynucleotide containing general codons or codons considering codon degeneracy in the polynucleotide.

Whether any two polynucleotide or polypeptide sequences have homology, similarity, or identity may be determined using known computer algorithms, such as the "FASTA" program using default parameters, for example, as in Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453), as performed in Needleman program of the EMBOSS package (EMBOSS: The European Molecular Biology Open Software Suite, Rice et al., 2000, Trends Genet. 16: 276-277) (version 5.0.0 or later version)(including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST from the National Center for Biotechnology Information Database, or ClustalW may be used to determine homology, similarity, or identity.

Homology, similarity, or identity of a polynucleotide or a polypeptide may be determined by comparing sequence information using a GAP computer program, for example, Needleman et al. (1970), J Mol Biol. 48:443, as disclosed, for example, in Smith and Waterman, Adv. Appl. Math (1981) 2:482. In summary, the GAP program may be defined as the value of dividing the number of similar aligned symbols (i.e., nucleotides or amino acids) by the total number of symbols in the shorter one of two sequences. Default parameters for the GAP program may comprise (1) a binary comparison matrix (containing values of 1 for identity and 0 for non-identity) and a weighted comparison matrix of Gribskov et al(1986) Nucl. Acids Res. 14: 6745, as disclosed by Schwartz and Dayhoff, eds., Atlas Of Protein Sequence And Structure, National Biomedical Research Foundation, pp. 353-358 (1979)(or EDNAFULL (EMBOSS version of NCBI NUC4.4) substitution matrix); (2) a penalty of 3.0 for each gap and an additional penalty of 0.10 for each symbol in each gap (or a gap opening penalty of 10, a gap extension penalty of 0.5); and (3) no penalty for a terminal gap.

One aspect provides a microorganism having enhanced activity of an aromatic amino acid transporter. The microorganism having the enhanced activity of the aromatic amino acid transporter may have one or more properties selected from: (i) increased valine production capability; (ii) increased sugar consumption rate; and (iii) decreased production capability of by-products (e.g., one or more amino acids selected from the group consisting of aromatic amino acids such as phenylalanine, tyrosine, and tryptophan), compared to a parent strain or wild-type microorganism without the enhanced activity.

The enhancement of the activity of the aromatic amino acid transporter may mean that the activity of the aromatic amino acid transporter is increased compared to the intrinsic activity. The enhancement may be used interchangeably with terms such as activation, up-regulation, overexpression, and increase. Here, activation, enhancement, up-regulation, overexpression, and increase may include both exhibiting activity not originally present, and exhibiting activity that is improved compared to the intrinsic activity or activity before modification. The "intrinsic activity" refers to the activity of a specific aromatic amino acid transporter originally possessed by a parent strain or a non-modified microorganism before modification, when a phenotypic change is caused by genetic mutation due to natural or artificial factors. This may be used interchangeably with "activity before modification". That the activity of the aromatic amino acid transporter is "enhanced," "up-regulated," "overexpressed," or "increased" compared to the intrinsic activity may refer to an increase in the activity of a specific aromatic amino acid transporter that the parent strain or non-modified microorganism originally possessed before modification, for example, an increase in the expression of the gene encoding the aromatic amino acid transporter. Whether the activity of the aromatic amino acid transporter is enhanced may be confirmed from the mRNA transcription level of the gene encoding the aromatic amino acid transporter, the expression level of the aromatic amino acid transporter, the activity level of the aromatic amino acid transporter, or the amount of by-products (e.g., aromatic amino acids) produced by the microorganism.

Various methods well known in the art may be applied to the enhancement of the activity of the aromatic amino acid transporter, and are not limited as long as they are capable of enhancing the activity of the aromatic amino acid transporter compared to a microorganism before modification. Specifically, gene engineering and/or protein engineering which are routine methods of molecular biology well known to those of ordinary skill in the art may be employed, but are not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide (aromatic amino acid transporter) of the present disclosure may be achieved by:
1) increase in intracellular copy number of a polynucleotide encoding the polypeptide;
2) replacement of a gene expression regulatory region of a gene encoding the polypeptide with a sequence having strong activity;
3) modification of the start codon of a gene transcript encoding the polypeptide or the nucleotide sequence encoding 5'-UTR region;
4) modification of an amino acid sequence of the polypeptide to enhance the polypeptide activity;
5) modification of a polynucleotide sequence encoding the polypeptide to enhance the polypeptide activity (e.g., modifying the polynucleotide sequence of the polypeptide gene to encode a polypeptide that is modified to enhance the activity of the polypeptide);
6) introducing a heterologous polypeptide exhibiting the activity of the polypeptide or a heterologous polynucleotide encoding the same;
7) codon optimization of a polynucleotide encoding the polypeptide;
8) modifying or chemically derivatizing a selected exposed site by analyzing a tertiary structure of the polypeptide; or
9) a combination of two or more selected from 1) to 8), but is not particularly limited thereto.

More specifically, the increase in the intracellular copy number of the polynucleotide encoding the polypeptide of item 1) may be achieved by introducing one copy or two or more copies of a vector comprising the polynucleotide encoding the polypeptide into a microorganism (host cell). The polynucleotide encoding the polypeptide may be introduced into the microorganism by a vector capable of inserting the polynucleotide into the chromosome of the microorganism, or in the form of a vector capable of replicating and functioning independently of the microorganism, but is not limited thereto.

The polynucleotide encoding the polypeptide may be operably linked to a promoter.

In the present disclosure, the term "operably linked" means that the promoter of the present disclosure is functionally linked to the gene sequence so as to initiate and mediate transcription of the target gene (e.g., a gene encoding the aromatic amino acid transporter). Operable linkage may be prepared using gene recombinant techniques known in the art, and site-specific DNA cleavage and ligation may be performed using cleavage and ligation enzymes, etc., known in the art of the present disclosure.

The promoter may be one or more selected from the group consisting of: an original promoter of the polynucleotide encoding the polypeptide; and a strong promoter different from the original promoter.

The original promoter may refer to a promoter operably linked to the polynucleotide encoding the polypeptide in the chromosome of a wild-type microorganism. In one embodiment, the original promoter may be a promoter linked to the NCgl1063 gene of *Corynebacterium glutamicum*.

The strong promoter may be a promoter of an endogenous gene or a heterologous gene of a microorganism. The strong promoter may be derived from a microorganism of the genus *Corynebacterium*. In one specific example, the strong promoter may be derived from *Corynebacterium glutamicum*.

In one embodiment, the strong promoter may be Ppyk (promoter of the pyk gene), Pald (promoter of the ald gene), PctaE (promoter of the ctaE gene), PpfkA (promoter of the pfkA gene), CJ1 to CJ7 promoters (Patent No. US 7662943 B2), lac promoter, trp promoter, trc promoter, tac promoter, lambda phage PR promoter, PL promoter, tet promoter, gapA promoter, SPL7 promoter, SPL13 (sm3) promoter (Patent No. US 10584338 B2), O2 promoter (Patent No. US 10273491 B2), tkt promoter, yccA promoter, or the like, but is not limited thereto.

The pyk gene may be a gene encoding pyruvate kinase. In one embodiment, the promoter of the pyk gene may comprise the nucleic acid sequence of SEQ ID NO: 3 or consist of the nucleic acid sequence of SEQ ID NO: 3.

The ald gene may be a gene encoding acetaldehyde dehydrogenase. In one embodiment, the promoter of the ald gene may comprise the nucleic acid sequence of SEQ ID NO: 4 or consist of the nucleic acid sequence of SEQ ID NO: 4.

The ctaE gene may be a gene encoding cytochrome c oxidase subunit 3. In one embodiment, the promoter of the ctaE gene may comprise the nucleic acid sequence of SEQ ID NO: 21 or consist of the nucleic acid sequence of SEQ ID NO: 21.

The pfkA gene may be a gene encoding ATP-dependent 6-phosphofructokinase isozyme 1. In one embodiment, the promoter of the pfkA gene may comprise the nucleic acid sequence of SEQ ID NO: 22 or consist of the nucleic acid sequence of SEQ ID NO: 22.

The microorganism may have enhanced activity of the aromatic amino acid transporter by comprising a gene encoding an aromatic amino acid transporter operably linked to the strong promoter.

The polynucleotide encoding the polypeptide may be inserted into a position that does not affect the expression of other genes of a host cell, for example, within a genomic safe harbor. In one embodiment, the genomic safe harbor may be a region between the NCgl0866 gene and the NCgl0867 gene of *Corynebacterium glutamicum*, or a region between the NCgl2195 gene and the NCgl2196 gene of *Corynebacterium glutamicum*.

The replacement of the gene expression regulatory region of a gene encoding the polypeptide with a sequence having strong activity of item 2) may be, for example, the generation of a mutation in a sequence by deletion, insertion, non-conservative or conservative substitution, or a combination thereof so as to further enhance the activity of the expression regulatory region or a replacement with a sequence having stronger activity. The expression regulatory region may comprise, but is not particularly limited to, a promoter, an operator sequence, a sequence encoding a ribosome binding site, and a sequence regulating termination of transcription and translation. For example, replacing the gene expression regulatory region of the gene encoding the aromatic amino acid transporter with a sequence having strong activity may be the replacement of the original promoter with a strong promoter, but is not limited thereto.

The microorganism having enhanced activity of an aromatic amino acid transporter may have enhanced activity of an aromatic amino acid transporter gene by operable linkage of a strong promoter to the gene encoding the aromatic amino acid transporter. As described above, that the strong promoter is operably linked to the aromatic amino acid transporter gene may mean that the original promoter of the aromatic amino acid transporter gene is replaced (substituted) with a strong promoter, or that the strong promoter is positioned in front of the aromatic amino acid transporter gene so that the expression of the aromatic amino acid transporter gene is regulated by the strong promoter.

The strong promoter may be one or more selected from the group consisting of Ppyk (promoter of the pyk gene), Pald (promoter of the ald gene), PctaE (promoter of the ctaE gene), PpfkA (promoter of the pfkA gene), CJ1 to CJ7 promoters (Patent No. US 7662943 B2), lac promoter, trp promoter, trc promoter, tac promoter, lambda phage PR promoter, PL promoter, tet promoter, gapA promoter, SPL7 promoter, SPL13 (sm3) promoter (Patent No. US 10584338 B2), O2 promoter (Patent No. US 10273491 B2), tkt promoter, yccA promoter, etc., as described above, but is not limited thereto.

The modification of the start codon of a gene transcript encoding the polypeptide or the nucleotide sequence encoding 5'-UTR region of item 3) may be, for example, a substitution with a nucleotide sequence encoding another start codon having a higher polypeptide expression level relative to the endogenous start codon, but is not limited thereto.

The modification of the amino acid sequence or the polynucleotide sequence of items 4) and 5) may be the generation of a mutation in the sequence by deletion, insertion, non-conservative or conservative substitution, or a combination thereof in the amino acid sequence of the polypeptide or the polynucleotide sequence encoding the polypeptide so as to enhance the activity of the polypeptide, or a replacement with an amino acid sequence or a polynucleotide sequence improved to have stronger activity or an amino acid sequence or a polynucleotide sequence improved to have increased activity, but is not limited thereto. The replacement may be performed specifically by inserting the polynucleotide into a chromosome by homologous recombination, but is not limited thereto. The vector used in this case may further include a selection marker for confirming insertion into the chromosome.

The introduction of a heterologous polynucleotide exhibiting the activity of the polypeptide of item 6) may be the introduction of a heterologous polynucleotide encoding a polypeptide exhibiting the same or similar activity as the polypeptide into a host cell. The heterologous polynucleotide is not limited by origin or sequence as long as it exhibits the same or similar activity as the polypeptide. A method used for the introduction may be performed by those skilled in the art by appropriately selecting a known transformation method, and the polypeptide is produced by expression of the introduced polynucleotide in the host cell, thereby increasing its activity.

The codon optimization of a polynucleotide encoding the polypeptide of item 7) may be codon optimization of an endogenous polynucleotide to increase transcription or translation within the host cell, or codon optimization of a heterologous polynucleotide to optimize transcription or translation within the host cell.

The modification or chemical derivatization of an exposed site by analyzing a tertiary structure of the polypeptide of item 8) may be, for example, performed by comparing sequence information of a polypeptide to be analyzed with sequence information of known proteins stored in a database to determine a template protein candidate based on a degree of sequence similarity and confirming a structure based thereon, and selecting an exposed site to be modified or chemically derivatized, and modifying or chemically derivatizing the same.

Such enhancement of polypeptide activity may be an increase in the activity or concentration expression level of the corresponding polypeptide relative to the activity or concentration of the polypeptide expressed in a wild-type microorganism or microorganism strain before modification, or an increase in the amount of a product produced from the corresponding polypeptide, but is not limited thereto.

In the microorganism of the present disclosure, modification of a part or all of the gene for enhancing the activity of the aromatic amino acid transporter may be induced by (a) homologous recombination using a chromosomal insertion vector into the microorganism or genome editing using engineered nucleases (e.g., CRISPR-Cas9), and/or (b) treatment with light, such as ultraviolet rays and radiation, and/or chemical substances, but is not limited thereto. The method for modifying part or all of the gene may include a method using DNA recombinant technology. For example, a nucleotide sequence or vector comprising a nucleotide sequence having homology with a target gene may be introduced into the microorganism to cause homologous recombination, thereby causing deletion of a part or all of the gene. The nucleotide sequence or vector to be introduced may comprise a dominant selection marker, but is not limited thereto.

The microorganism of the present disclosure may be a microorganism having enhanced activity of an aromatic amino acid transporter, or a microorganism genetically modified through a vector to enhance the activity of an aromatic amino acid transporter (e.g., a recombinant microorganism), but is not limited thereto. The microorganism (or strain, recombinant cell) of the present disclosure may be a microorganism having enhanced activity of the aromatic amino acid transporter, thereby having valine production capability or improved valine production capability (or production amount).

The microorganism of the present disclosure may have enhanced activity of an aromatic amino acid transporter and may have one or more properties selected from: (i) increased valine production capability; (ii) increased sugar consumption rate; and (iii) decreased production capability of by-products (e.g., one or more amino acids selected from the group consisting of aromatic amino acids such as phenylalanine, tyrosine, and tryptophan).

The microorganism of the present disclosure may be a microorganism that naturally has valine production capability, or a microorganism that has valine production capability conferred or improved on a parent strain without valine production capability, but is not limited thereto. The microorganism may be a microorganism having valine production capability or increased valine production capability. The microorganism may be a microorganism having valine production capability conferred or improved with valine by introducing enhancement of an aromatic amino acid transporter into a microorganism without valine production capability, or into a microorganism having valine production capability, but is not limited thereto.

That the microorganism has valine production capability or improved valine production capability may indicate that the microorganism has valine production capability conferred, unlike a non-modified microorganism, a cell before recombination, a parent strain, and/or a wildtype strain that does not have valine production capability, or that the valine production capability is increased compared to a non-modified microorganism, a cell before recombination, a parent strain, and/or a wild-type strain.

The microorganism having enhanced activity of an aromatic amino acid transporter according to one example may have increased valine production capability compared to the microorganism before introduction, that is, the non-modified microorganism of the same species. In the present disclosure, the term "non-modified microorganism" does not exclude a strain comprising a mutation that may occur naturally in a microorganism, and may refer to a wild-type strain or a natural strain itself, or a strain before transformation caused by genetic mutation due to natural or artificial factors. For example, the non-modified microorganism may refer to a microorganism in which the activity of the aromatic amino acid transporter is not enhanced or before enhancement of the activity of the aromatic amino acid transporter. The "non-modified microorganism" may be used interchangeably with "pre-modification strain", "pre-modification microorganism", "non-variant strain", "non-modified strain", "non-variant microorganism" or "reference microorganism".

The microorganism (or strain, recombinant cell) may further comprise mutations that increase valine production, and the position of the mutation and/or the type of gene and/or protein that is the target of the mutation may be included without limitation as long as it increases valine production. The recombinant cell may be used without limitation as long as it is a cell capable of transformation.

In one embodiment, the microorganism of the present disclosure may further comprise an A42V variant of the acetolactate synthase isozyme 1 small subunit (IlvN) protein or a nucleotide encoding the same (see Biotechnology and Bioprocess Engineering, June 2014, Volume 19, Issue 3, pp 456-467).

In one embodiment, the non-modified microorganism, which is a target strain for comparing the increase in the valine production capability, may be a *Corynebacterium glutamicum* ATCC13032 strain, *Corynebacterium glutamicum* KCCM11201P (Patent No. US 8465962 B), or a strain having improved valine production capability by introduction of an A42V mutation of the acetolactate synthase isozyme 1 small subunit (IlvN) protein in *Corynebacterium glutamicum* ATCC14067 [ilvN (A42V); Biotechnology and Bioprocess Engineering, June 2014, Volume 19, Issue 3, pp 456-467; Patent No. US 11180784 B2], but is not limited thereto.

In one embodiment, the microorganism (or strain, recombinant cell) having improved (increased) valine production capability (or production amount, yield) may have a valine production capability increased by 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, or 35% or more (the upper limit is not particularly limited, and may be, for example, about 200% or less, about 150% or less, about 100% or less, about 50% or less, about 45% or less, about 40% or less, or about 35% or less), compared to the parent strain before mutation or the non-modified microorganism, but is not limited thereto.

In another example, the microorganism (or strain, recombinant cell) having improved (increased) valine production capability (or production amount, yield) may have a valine production capability increased by about 1.05 times or more, about 1.1 times or more, about 1.15 times or more, about 1.2 times or more, about 1.25 times or more, or about 1.3 times or more, compared to the parent strain before mutation or the non-modified microorganism, but is not limited thereto. The term "about" encompasses all ranges including ±0.5, ±0.4, ±0.3, ±0.2, ±0.1, etc., and includes all ranges of values equal to or similar to the value following the term "about," but is not limited thereto.

The microorganism may be a microorganism of the genus *Corynebacterium*.

The microorganism of the genus *Corynebacterium* may be one or more selected from the group consisting of *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium ammoniagenes*, *Corynebacterium pollutisoli*, *Corynebacterium imitans*, *Corynebacterium testudinoris*, *Corynebacterium acetoacidophilum*, *Corynebacterium acetoglutamicum*, *Corynebacterium alkanolyticum*, *Corynebacterium lilium*, *Corynebacterium melassecola*, *Corynebacterium thermoaminogenes*, *Corynebacterium herculis*, and *Corynebacterium flavescens*, but is not limited thereto.

Another aspect provides a gene expression cassette comprising the promoter and an aromatic amino acid transporter gene operably linked to the promoter.

In the present disclosure, the term "gene expression cassette" may refer to a unit cassette that comprises a promoter and a target gene and is capable of expressing a target gene operatively linked downstream of the promoter. Various factors that facilitate efficient expression of the target gene may be comprised inside or outside of such gene expression cassette. The gene expression cassette may comprise a transcription termination signal, a ribosome binding site, and a translation termination signal in addition to the promoter that is operably linked to the target gene, but is not limited thereto.

The term "target gene", for the object of the present disclosure, refers to a gene of which expression is regulated by the promoter sequence of the present disclosure. The protein encoded by the target gene may be expressed as a "target protein," and the gene encoding the "target protein" may be expressed as a "target gene".

In one embodiment, the target gene may refer to an aromatic amino acid transporter gene to be expressed through the promoter.

The gene encoding the aromatic amino acid transporter may be derived from a microorganism of the genus *Corynebacterium*, as described above. The gene encoding the aromatic amino acid transporter may be derived from *Corynebacterium glutamicum*. The gene encoding the aromatic amino acid transporter may comprise the nucleic acid sequence of SEQ ID NO: 2 or may consist of the nucleic acid sequence of SEQ ID NO: 2. In one embodiment, the aromatic amino acid transporter gene may have 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more homology or identity with the nucleic acid sequence of SEQ ID NO: 2, comprise the nucleic acid sequence, or consist of the nucleic acid sequence, but is not limited thereto.

The promoter may be derived from a microorganism of the genus *Corynebacterium*, as described above. In one embodiment, the promoter may be derived from *Corynebacterium glutamicum*.

The promoter may be one or more strong promoters selected from the group consisting of Ppyk (promoter of the pyk gene), Pald (promoter of the ald gene), PctaE (promoter of the ctaE gene), PpfkA (promoter of the pfkA gene), CJ1 to CJ7 promoters (Patent No. US 7662943 B2), lac promoter, trp promoter, trc promoter, tac promoter, lambda phage PR promoter, PL promoter, tet promoter, gapA promoter, SPL7 promoter, SPL13 (sm3) promoter (Patent No. US 10584338 B2), O2 promoter (Patent No. US 10273491 B2), tkt promoter, yccA promoter, etc., as described above, but is not limited thereto.

In one embodiment, the promoter may be one or more selected from the group consisting of a promoter of the pyk gene (SEQ ID NO: 3) and a promoter of the ald gene (SEQ ID NO: 4), but is not limited thereto.

In one embodiment, the gene expression cassette may comprise: one or more promoters selected from the group consisting of a promoter of a pyk gene and a promoter of an ald gene; and
a gene encoding an aromatic amino acid transporter operably linked to the promoter. The microorganism may be a microorganism comprising the gene expression cassette.

Still another aspect provides a composition for producing valine, comprising one or more selected from the group consisting of the microorganism and a medium in which the microorganism is cultured. The composition of the present disclosure may further comprise any appropriate excipient generally used in the composition for producing valine, and such an excipient may be, for example, a preservative agent, a wetting agent, a dispersing agent, a suspending agent, a buffering agent, a stabilizing agent, or an isotonic agent, but is not limited thereto.

In the composition of the present disclosure, the microorganism (strain), medium, and valine, etc. are as described in the other aspects above.

Still another aspect provides a use of the microorganism and one or more selected from the group consisting of the microorganism and a medium in which the microorganism is cultured, for producing valine.

Yet another aspect provides a method for producing valine or a method for increasing valine production, comprising culturing the microorganism in a medium. The microorganism and valine are as described above.

In the present disclosure, the term "culture" may refer to growing the microorganism of the present disclosure (e.g., a *Corynebacterium glutamicum* strain) under appropriately controlled environmental conditions. The culture process of the present disclosure may be performed according to an appropriate medium and culture conditions known in the art. Such a culturing process may be easily adjusted and used by those skilled in the art according to a selected strain. Specifically, culturing may be performed in a batch manner, a continuous manner, and/or a fed-batch manner, but is not limited thereto.

In the present disclosure, the term "medium" refers to a material mixed with, as a main ingredient, nutrients required for culturing the microorganism of the present disclosure (e.g., a *Corynebacterium glutamicum* strain), and supplies nutrients and growth factors, including water essential for survival and growth. Specifically, the medium and other culture conditions used for culturing the microorganism of the present disclosure may be used without any special limitation as long as they are a medium used for conventional culturing of a microorganism, but the microorganism of the present disclosure may be cultured in a conventional medium containing an appropriate carbon source, nitrogen source, phosphorus source, inorganic compound, amino acid and/or vitamin, etc. under an aerobic condition while controlling temperature, pH, etc.

In one embodiment, the culture medium for a strain of the genus *Corynebacterium* may be found in the literature entitled ["Manual of Methods for General Bacteriology" by the American Society for Bacteriology (Washington D.C., USA, 1981)].

In the present disclosure, the carbon source may include carbohydrates such as glucose, saccharose, lactose, fructose, sucrose, and maltose; sugar alcohols such as mannitol and sorbitol; organic acids such as pyruvic acid, lactic acid, and citric acid; amino acids such as glutamic acid, methionine, and lysine. In addition, natural organic nutrients such as starch hydrolysate, molasses, blackstrap molasses, rice bran, cassava, sugarcane bagasse, and corn steep liquor may be used. Specifically, carbohydrates such as glucose and sterilized, pretreated molasses (i.e., molasses converted to reducing sugars) may be used, and various carbon sources may be used without limitation in appropriate amounts. These carbon sources may be used alone or in a combination of two or more, but are not limited thereto.

Inorganic nitrogen sources such as ammonia, ammonium sulfate, ammonium chloride, ammonium acetate, ammonium phosphate, ammonium carbonate, and ammonium nitrate; and organic nitrogen sources such as amino acids (e.g., glutamic acid, methionine, and glutamine); peptone, NZ-amine, meat extract, yeast extract, malt extract, corn steep liquor, casein hydrolysate, fish or its decomposition products thereof, and defatted soybean cake or its decomposition products thereof may be used as the nitrogen source. These nitrogen sources may be used alone or in a combination of two or more, but are not limited thereto.

The phosphorus source may include monopotassium phosphate, dipotassium phosphate, or corresponding sodium-containing salts thereof, etc. Sodium chloride, calcium chloride, iron chloride, magnesium sulfate, iron sulfate, manganese sulfate, and calcium carbonate, etc. may be used as the inorganic compound, and amino acids, vitamins, and/or appropriate precursors may be included. These components or precursors may be added to the medium in a batch manner or a continuous manner, but are not limited thereto.

In addition, during culturing of the microorganism of the present disclosure, the pH of the medium may be adjusted by adding compounds such as ammonium hydroxide, potassium hydroxide, ammonia, phosphoric acid, sulfuric acid to the medium in an appropriate manner. In addition, during culturing, an antifoaming agent, such as fatty acid polyglycol ester, may be used to suppress foam generation. In addition, oxygen or oxygen-containing gas may be injected into the medium in order to maintain an aerobic state in the medium, or nitrogen, hydrogen, or carbon dioxide gas may be injected without gas injection in order to maintain an anaerobic or microaerobic state in the medium, but is not limited thereto.

In the culturing of the present disclosure, the culturing temperature may be maintained at 20 to 45 °C, specifically 25 to 40 °C, and the culturing may be performed for about 10 to 160 hours, but is not limited thereto.

Valine produced by the culturing of the present disclosure may be secreted into the medium or remain within a cell.

The method for producing valine or the method for increasing valine production of the present disclosure may further comprise preparing the microorganism (strain) of the present disclosure, preparing a medium for culturing the microorganism, or a combination thereof (in any order), for example, before the culturing step.

The method for producing valine or increasing valine production of the present disclosure may further comprise recovering valine from the medium resulting from the culturing (a medium in which culturing has been performed) or from the microorganism (a strain of the genus *Corynebacterium*). The recovery step may be further comprised after the culturing step.

The recovery may involve collecting desired valine using an appropriate method known in the art according to the culturing method of the microorganism of the present disclosure, for example, batch, continuous, or fed-batch culturing methods. For example, centrifugation, filtration, crystallization, treatment with a protein precipitating agent (salting out method), extraction, ultrasonic disruption, ultrafiltration, dialysis, various types of chromatography such as molecular sieve chromatography (gel filtration), adsorption chromatography, ion exchange chromatography, affinity chromatography, HPLC or a combination thereof may be used, and the desired valine may be recovered from the medium or the microorganism using an appropriate method known in the art.

In addition, the method for producing valine or the method for increasing valine production of the present disclosure may further comprise a purification step. The purification may be performed using an appropriate method known in the art. In one embodiment, when the method for producing valine or the method for increasing valine production of the present disclosure comprises both the recovery step and the purification step, the recovery step and the purification step may be performed continuously or discontinuously regardless of the order, or may be performed simultaneously or integrated into a single step, but are not limited thereto.

### [Advantageous Effects]

A microorganism having enhanced activity of an aromatic amino acid transporter of the present disclosure has excellent valine production capability, and thus can be efficiently utilized for mass production of valine.

### [Embodiment of Invention]

Hereinafter, the present invention will be described in more detail with reference to the following Examples. However, these are only intended to exemplify the present invention, and the scope of the present invention is not limited by these Examples.

### Example 1. Preparation of plasmid for enhancing aroP

A plasmid that enhances the activity of aroP, an aromatic amino acid transporter, was prepared by additionally inserting an aroP gene into the chromosome based on the previously known valine-producing strain *Corynebacterium glutamicum* KCCM11201P (Patent No. US 8465962 B). The additionally inserted aroP was designed to be expressed by two types of promoters.

**[Table 1]**

| SEQ ID NO: | Name | Sequence |
|---|---|---|
| **1** | **aroP** | MAKSNEGLGTGLRTRHLTMMGLGSAIGAGLFLGTGVGI RAAGPAVLLAYIIAGAIVVLVMQMLGEMAAARPASGSFSR YGEDAFGHWAGFSLGWLYWFMLIMVMGAEMTGAAAIM GAWFGVEPWIPSLVCVVFFAVVNLVAVRGFGEFEYWFAF IKVAVIIAFLIIGIALIFGWLPGSTFVGTSNFIGDHGFMPNGI SGVAAGLLAVAFAFGGIEIVTIAAAESDKPREAISLAVRAVI WRISVFYLGSVLVITFLMPYESINGADTAAESPFTQILAMA NIPGTVGFMEAIIVLALLSAFNAQIYATSRLVFSMANRQDA PRVFSKLSTSHVPTNAVLLSMFFAFVSVGLQYWNPAGLL DFLLNAVGGCLIVVWAMITLSQLKLRKELQANGEISTVRM WAHPWLGILTLVLLAGLVALMLGDAASRSQVYSVAIVYGF LVLLSFVTVNSPLRGGRTPSDLN |
| | amino acid sequence | |
| **2** | **aroP** | ATGGCTAAATCTAATGAAGGGCTGGGAACCGGACTTC GGACCCGCCACCTCACAATGATGGGACTCGGCTCCG CAATTGGTGCCGGACTGTTCCTCGGCACCGGCGTTG GTATCCGCGCAGCTGGCCCCGCAGTGCTCCTGGCGT ACATCATCGCCGGAGCCATCGTTGTGCTTGTTATGCAA ATGCTCGGCGAGATGGCTGCTGCCCGTCCCGCCTCC GGATCGTTTTCACGTTACGGCGAGGATGCTTTCGGCC ACTGGGCTGGTTTCTCCCTCGGTTGGTTGTACTGGTT CATGCTGATTATGGTGATGGGCGCCGAAATGACTGGC GCTGCTGCCATCATGGGTGCATGGTTCGGCGTCGAAC CGTGGATTCCTTCGCTTGTCTGCGTGGTCTTCTTCGC TGTGGTGAACCTCGTCGCGGTTCGCGGTTTCGGTGA ATTCGAGTACTGGTTCGCATTCATTAAGGTCGCGGTGA TCATCGCTTTCCTCATCATTGGTATTGCTCTTATTTTCG GATGGCTTCCCGGATCCACCTTTGTGGGAACCTCAAA CTTCATCGGTGATCACGGATTCATGCCCAATGGTATTT CTGGTGTTGCTGCTGGTTTGCTCGCGGTGGCTTTTGC CTTTGGTGGCATTGAAATTGTCACCATTGCAGCTGCAG AGTCCGATAAGCCACGTGAAGCTATTTCCCTGGCGGT GCGTGCCGTGATTTGGCGTATTTCAGTCTTTTACTTGG GCTCTGTTTTGGTCATCACTTTCCTCATGCCTTATGAG TCGATCAATGGTGCCGACACCGCTGCGGAATCCCCCT TCACCCAAATCCTGGCGATGGCAAACATCCCTGGCAC GGTTGGTTTCATGGAAGCGATCATCGTTCTAGCACTGC TTTCCGCTTTCAACGCCCAAATCTATGCCACTTCTCGT TTGGTATTTTCCATGGCGAATCGACAAGACGCTCCACG AGTTTTCAGTAAGCTCAGCACCAGCCACGTCCCCACC AATGCGGTGCTGTTGTCCATGTTCTTCGCCTTCGTTTC AGTTGGTTTGCAGTACTGGAATCCAGCCGGTCTGCTT GATTTCCTCCTCAACGCTGTTGGCGGTTGTTTGATTGT GGTGTGGGCAATGATCACTTTGTCACAGTTGAAACTC CGCAAAGAACTGCAAGCAAACGGTGAAATCTCCACGG TTCGCATGTGGGCGCACCCATGGTTAGGCATCCTCAC CTTGGTTTTGTTGGCAGGACTCGTTGCTCTCATGCTC GGCGACGCAGCCTCCCGCAGCCAGGTTTACTCAGTG GCAATTGTGTACGGATTCTTGGTTCTTTTGTCCTTCGT CACAGTTAACAGCCCATTGCGCGGAGGTCGCACCCCT TCCGACTTGAACTGA |
| | nucleotide sequence | |
| **3** | **Ppyk** | CTCTACGTAGCTGGTTACACCTTGGGCCGTTTCTGGAT TGAACAAATGCGCGTTGATGAAGCCACGCTTATTGGC GGCATCCGAATCAACACCATCGTCTCCGCAGTAGTGT TTGCCGGCGCGATCATTGTGTTCTTCCTGTTGAAGAA GGGTAGGGAAACTCCCGAAGAGGTAGATCCGACTTTC ACAGCGTCTGTTGCAGCAGATGCTGTAGCTTCGCCAG ATGGAAAACCCTTGCCGAAAGCAGAGGAGGGCATTGA TGGAGAAACGCCCTCAACGCGATAGGTTTCAACCATA GGCCTGACCTGGCTGAGATGTTTTTGGTAGAAAAACC GAGTGCCCGAATTGTTTTGTGGGTGCCCGGTTTTTCT GATTTAAGCACGTCAGAGGCGTAGAACATTGTCTGTTC ACACTCTGGGTCGCAAGATTCATCGAGAATTAATGGTA GTATCTGTGGCTTGAGGGGGAATGACGTACTAGGCTT ATGGGC |
| | promoter nucleotide sequence | |
| **4** | **Pald** | CGATCATCGAACTCGGCGAAGAAAACCGAAACCTCAA AGAATCCCTGCGTAAGGTCACAGCTGAGAATGAGCAG CTCAAAGATCAATTACGCAGCGGGCGTCCGCGTGGC GAGCTGGTGCACGTGCCCCGCTCCACCGCGGTGGTC ATGTGGGAACGCCGCAAGGGGCGTTCCAAGTAAAAAC ATGCTTGTCGACGCCACTTTCTAGCAAATTAAGCGGGC ACCTCCATTTATCTTTTGGAGGTGCCCGTTTCGTGCTT TCGCCAATTAGATACATGGATAACCACCCGAACAGGGG GTAATAACTTTTGAAAGGCTTTCGGCGTTGAGCTGCGA GAATTTTGAGAAAAAGGGGGTGAATTTAACAGGGGTT CTAGCGCGGATTGATTTTCGTGAATATGGTGGCTGCTA AGCGTGCGAATGTGCGCGTTATCACAATCGTTGACCA AGTGTCACCTGACGCACAGGTAGTGCTCAGGTGGAG GTGGCCCAAAGGAGACCCA |
| | promoter nucleotide sequence | |

### Example 1-1. Preparation of plasmid for gene insertion

In order to enhance a target gene by additionally inserting an aroP gene into the chromosome of *Corynebacterium glutamicum* KCCM11201P, an intergenic site that does not encode genetic information in the KCCM11201P genome was used as an insertion site to eliminate influence by gene deletion, and the non-coding region between NCgl0866 and NCgl0867 (hereinafter represented as NCgl0867up) was selected. In this case, the Ppyk (SEQ ID NO: 3) and Pald (SEQ ID NO: 4) promoters, which are stronger promoters than the promoter of the endogenous aroP gene, were used. Template chromosomes for the experiment were all subjected to PCR using the KCCM11201P genome. PfuUltraTM high-fidelity DNA polymerase (Stratagene) was used as a polymerase for the PCR reaction, and the PCR conditions consist of denaturation at 95 °C for 30 seconds; denaturation at 55 °C for 30 seconds; and polymerization reaction at 72°C for 1 minute, wherein the denaturation, annealing, and polymerization reaction under these conditions were repeated 28 times.

Fragment A of 819 bp in the 5' upstream region of NCgl0867up was obtained using the primers of SEQ ID NO: 5 and SEQ ID NO: 6, fragment B of 832 bp in the 3' downstream region of NCgl0867up was obtained using the primers of SEQ ID NO: 9 and SEQ ID NO: 10, and fragment C of 1430 bp comprising the ORF portion of aroP and a part of the 3' downstream region of aroP was obtained using the primers of SEQ ID NO: 7 and SEQ ID NO: 8. Fragment E of 525 bp of the Ppyk promoter region was obtained using SEQ ID NO: 11 and SEQ ID NO: 12, and fragment F of 536 bp at the Pald promoter region was obtained using SEQ ID NO: 13 and SEQ ID NO: 14, respectively. The primer sequences used to perform each PCR are as shown in Table 2 below. The amplified products were purified using a PCR purification kit (QUIAGEN) and used as insert DNA fragments for vector preparation.

**[Table 2]**

| SEQ ID NO: | Name | Sequence |
|---|---|---|
| 5 | Primer | gctcggtacccggggatccTCATTCGTTCATAATTGATCCCACAG |
| 6 | Primer | GGGGAGTGAATGCTGGGTG |
| 7 | Primer | ATGGCTAAATCTAATGAAGGGCTG |
| 8 | Primer | TGGAGGGTCGGACGTCCAGAATAATTTCTATCGG |
| 9 | Primer | TCTGGACGTCCGACCCTCCACGCTGGA |
| 10 | Primer | cctgcaggtcgactctagaGGGCTCCTTCGTCATCATC |
| 11 | Primer | CCCAGCATTCACTCCCCCTCTACGTAGCTGGTTACACC |
| 12 | Primer | TTCATTAGATTTAGCCATGCCCATAAGCCTAGTACGTCA |
| 13 | Primer | CCCAGCATTCACTCCCCCGATCATCGAACTCGGCGAA |
| 14 | Primer | TCATTAGATTTAGCCATTGGGTCTCCTTTGGGCCAC |

The vector pDC24_ΔNCgl0867up::Ppyk_aroP capable of inserting the aroP gene expressed under the Ppyk promoter into a position between NCgl0866 and NCgl0867 of KCCM11201P was prepared by cloning the previously obtained fragments A, B, C, E and the pDC24 vector (SEQ ID NO: 23, Table 3) treated with BamHI and XbaI (New England Biolabs, Beverly, MA) using the infusion cloning kit from TaKaRa according to the provided manual.

The vector pDC24_ΔNCgl0867up::Pald_aroP capable of inserting the aroP gene expressed under the Pald promoter into a position between NCgl0866 and NCgl0867 of KCCM11201P was prepared by cloning the previously obtained fragments A, B, C, F and the pDC24 vector treated with BamHI and XbaI (New England Biolabs, Beverly, MA) using the infusion cloning kit from TaKaRa according to the provided manual.

**[Table 3]**

| |
|---|
| GTTCGCTTGCTGTCCATAAAACCGCCCAGTCTAGCTATCGCCATGTAAGCCCACTGC AAGCTACCTGCTTTCTCTTTGCGCTTGCGTTTTCCCTTGTCCAGATAGCCCAGTAGCT GACATTCATCCGGGGTCAGCACCGTTTCTGCGGACTGGCTTTCTACGTGTTCCGCTT CCTTTAGCAGCCCTTGCGCCCTGAGTGCTTGCGGCAGCGTGAAGCTAGCTTTTATC GCCATTCGCCATTCAGGCTGCGCAACTGTTGGGAAGGGCGATCGGTGCGGGCCTC TTCGCTATTACGCCAGCTGGCGAAAGGGGGATGTGCTGCAAGGCGATTAAGTTGGG TAACGCCAGGGTTTTCCCAGTCACGACGTTGTAAAACGACGGCCAGTGAATTCGAG CTCGGTACCCGGGGATCCTCTAGAGTCGACCTGCAGGCATGCAAGCTTGGCGTAAT CATGGTCATAGCTGTTTCCTGTGTGAAATTGTTATCCGCTCACAATTCCACACAACATA CGAGCCGGAAGCATAAAGTGTAAAGCCTGGGGTGCCTAATGAGTGAGCTAACTCAC ATTAATTGCGTTGCGCTCACTGCCCGCTTTCCAGTCGGGAAACCTGTCGTGCCAGCT GCATTAATGAATCGGCCAACGCGCGGGGAGAGGCGGTTTGCGTATTGGGCGCTCTT CCGCTTCCTCGCTCACTGACTCGCTGCGCTCGGTCGTTCGGCTGCGGCGAGCGGT ATCAGCTCACTCAAAGGCGGTAATACGGTTATCCACAGAATCAGGGGATAACGCAGG AAAGAACATGTGAGCAAAAGGCCAGCAAAAGGCCAGGAACCGTAAAAAGGCCGCGT TGCTGGCGTTTTTCCATAGGCTCCGCCCCCCTGACGAGCATCACAAAAATCGACGCT CAAGTCAGAGGTGGCGAAACCCGACAGGACTATAAAGATACCAGGCGTTTCCCCCT GGAAGCTCCCTCGTGCGCTCTCCTGTTCCGACCCTGCCGCTTACCGGATACCTGTC CGCCTTTCTCCCTTCGGGAAGCGTGGCGCTTTCTCAATGCTCACGCTGTAGGTATCT CAGTTCGGTGTAGGTCGTTCGCTCCAAGCTGGGCTGTGTGCACGAACCCCCCGTTC AGCCCGACCGCTGCGCCTTATCCGGTAACTATCGTCTTGAGTCCAACCCGGTAAGAC ACGACTTATCGCCACTGGCAGCAGCCACTGGTAACAGGATTAGCAGAGCGAGGTAT GTAGGCGGTGCTACAGAGTTCTTGAAGTGGTGGCCTAACTACGGCTACACTAGAAG GACAGTATTTGGTATCTGCGCTCTGCTGAAGCCAGTTACCTTCGGAAAAAGAGTTGG TAGCTCTTGATCCGGCAAACAAACCACCGCTGGTAGCGGTGGTTTTTTTGTTTGCAA GCAGCAGATTACGCGCAGAAAAAAAGGATCTCAAGAAGATCCTTTGATCTTTTCTACG GGGTCTGACGCTCAGTGGAACGAAAACTCACGTTAAGGGATTTTGGTCATGAGATTA TCAAAAAGGATCTTCACCTAGATCCTTTTGGGGTGGGCGAAGAACTCCAGCATGAGA TCCCCGCGCTGGAGGATCATCCAGCCCTGATAGAAACAGAAGCCACTGGAGCACCT CAAAAACACCATCATACACTAAATCAGTAAGTTGGCAGCATCACCCGACGCACTTTGC GCCGAATAAATACCTGTGACGGAAGATCACTTCGCAGAATAAATAAATCCTGGTGTCC CTGTTGATACCGGGAAGCCCTGGGCCAACTTTTGGCGAAAATGAGACGTTGATCGG CACGTAAGAGGTTCCAACTTTCACCATAATGAAATAAGATCACTACCGGGCGTATTTT TTGAGTTATCGAGATTTTCAGGAGCTGATAGAAACAGAAGCCACTGGAGCACCTCAA AAACACCATCATACACTAAATCAGTAAGTTGGCAGCATCACCCGACGCACTTTGCGC |
| CGAATAAATACCTGTGACGGAAGATCACTTCGCAGAATAAATAAATCCTGGTGTCCCT GTTGATACCGGGAAGCCCTGGGCCAACTTTTGGCGAAAATGAGACGTTGATCGGCA CGTAAGAGGTTCCAACTTTCACCATAATGAAATAAGATCACTACCGGGCGTATTTTTT GAGTTATCGAGATTTTCAGGAGCTCTTTGGCATCGTCTCTCGCCTGTCCCCTCAGTT CAGTAATTTCCTGCATTTGCCTGTTTCCAGTCGGTAGATATTCCACAAAACAGCAGGG AAGCAGCGCTTTTCCGCTGCATAACCCTGCTTCGGGGTCATTATAGCGATTTTTTCG GTATATCCATCCTTTTTCGCACGATATACAGGATTTTGCCAAAGGGTTCGTGTAGACTT TCCTTGGTGTATCCAACGGCGTCAGCCGGGCAGGATAGGTGAAGTAGGCCCACCCG CGAGCGGGTGTTCCTTCTTCACTGTCCCTTATTCGCACCTGGCGGTGCTCAACGGG AATCCTGCTCTGCGAGGCTGGCCGGCTACCGCCGGCGTAACAGATGAGGGCAAGC GGATGGCTGATGAAACCAAGCCAACCAGGAAGGGCAGCCCACCTATCAAGGTGTAC TGCCTTCCAGACGAACGAAGAGCGATTGAGGAAAAGGCGGCGGCGGCCGGCATGA GCCTGTCGGCCTACCTGCTGGCCGTCGGCCAGGGCTACAAAATCACGGGCGTCGT GGACTATGAGCACGTCCGCGAGGGCGTCCCGGAAAACGATTCCGAAGCCCAACCTT TCATAGAAGGCGGCGGTGGAATCGAAATCTCGTGATGGCAGGTTGGGCGTCGCTTG GTCGGTCATTTCGAAAAAGGTTAGGAATACGGTTAGCCATTTGCCTGCTTTTATATAGT TCATATGGGATTCACCTTTATGTTGATAAGAAATAAAAGAAAATGCCAATAGGATATCG GCATTTTCTTTTGCGTTTTTATTTGTTAACTGTTAATTGTCCTTGTTCAAGGATGCTGT CTTTGACAACAGATGTTTTCTTGCCTTTGATGTTCAGCAGGAAGCTCGGCGCAAACG TTGATTGTTTGTCTGCGTAGAATCCTCTGTTTGTCATATAGCTTGTAATCACGACATTG TTTCCTTTCGCTTGAGGTACAGCGAAGTGTGAGTAAGTAAAGGTTACATCGTTAGGC GGATCAAGATCCATTTTTAACACAAGGCCAGTTTTGTTCAGCGGCTTGTATGGGCCA GTTAAAGAATTAGAAACATAACCAAGCATGTAAATATCGTTAGACGTAATGCCGTCAAT CGTCATTTTTGATCCGCGGGAGTCAGTGAACAGGTACCATTTGCCGTTCATTTTAAAG ACGTTCGCGCGTTCAATTTCATCTGTTACTGTGTTAGATGCAATCAGCGGTTTCATCA CTTTTTTCAGTGTGTAATCATCGTTTAGCTCAATCATACCGAGAGCGCCGTTTGCTAA CTCAGCCGTGCGTTTTTTATCGCTTTGCAGAAGTTTTTGACTTTCTTGACGGAAGAAT GATGTGCTTTTGCCATAGTATGCTTTGTTAAATAAAGATTCTTCGCCTTGGTAGCCATC TTCAGTTCCAGTGTTTGCTTCAAATACTAAGTATTTGTGGCCTTTATCTTCTACGTAGT GAGGATCTCTCAGCGTATGGTTGTCGCCTGAGCTGTAGTTGCCTTCATCGATGAACT GCTGTACATTTTGATACGTTTTTCCGTCACCGTCAAAGATTGATTTATAATCCTCTACA CCGTTGATGTTCAAAGAGCTGTCTGATGCTGATACGTTAACTTGTGCAGTTGTCAGT GTTTGTTTGCCGTAATGTTTACCGGAGAAATCAGTGTAGAATAAACGGATTTTTCCGT CAGATGTAAATGTGGCTGAACCTGACCATTCTTGTGTTTGGTCTTTTAGGATAGAATC ATTTGCATCGAATTTGTCGCTGTCTTTAAAGACGCGGCCAGCGTTTTTCCAGCTGTC |
| AATAGAAGTTTCGCCGACTTTTTGATAGAACATGTAAATCGATGTGTCATCCGCATTTT TAGGATCTCCGGCTAATGCAAAGACGATGTGGTAGCCGTGATAGTTTGCGACAGTGC CGTCAGCGTTTTGTAATGGCCAGCTGTCCCAAACGTCCAGGCCTTTTGCAGAAGAG ATATTTTTAATTGTGGACGAATCAAATTCAGAAACTTGATATTTTTCATTTTTTTGCTGTT CAGGGATTTGCAGCATATCATGGCGTGTAATATGGGAAATGCCGTATGTTTCCTTATAT GGCTTTTGGTTCGTTTCTTTCGCAAACGCTTGAGTTGCGCCTCCTGCCAGCAGTGC GGTAGTAAAGGTTAATACTGTTGCTTGTTTTGCAAACTTTTTGATGTTCATCGTTCATG TCTCCTTTTTTATGTACTGTGTTAGCGGTCTGCTTCTTCCAGCCCTCCTGTTTGAAGA TGGCAAGTTAGTTACGCACAATAAAAAAAGACCTAAAATATGTAAGGGGTGACGCCAA AGTATACACTTTGCCCTTTACACATTTTAGGTCTTGCCTGCTTTATCAGTAACAAACCC GCGCGATTTACTTTTCGACCTCATTCTATTAGACTCTCGTTTGGATTGCAACTGGTCT ATTTTCCTCTTTTGTTTGATAGAAAATCATAAAAGGATTTGCAGACTACGGGCCTAAAG AACTAAAAAATCTATCTGTTTCTTTTCATTCTCTGTATTTTTTATAGTTTCTGTTGCATGG GCATAAAGTTGCCTTTTTAATCACAATTCAGAAAATATCATAATATCTCATTTCACTAAAT AATAGTGAACGGCAGGTATATGTGATGGGTTAAAAAGGATCACCCCAGAGTCCCGCT CAGAAGAACTCGTCAAGAAGGCGATAGAAGGCGATGCGCTGCGAATCGGGAGCGG CGATACCGTAAAGCACGAGGAAGCGGTCAGCCCATTCGCCGCCAAGCTCTTCAGCA ATATCACGGGTAGCCAACGCTATGTCCTGATAGCGGTCCGCCACACCCAGCCGGCC ACAGTCGATGAATCCAGAAAAGCGGCCATTTTCCACCATGATATTCGGCAAGCAGGC ATCGCCATGGGTCACGACGAGATCCTCGCCGTCGGGCATCCGCGCCTTGAGCCTG GCGAACAGTTCGGCTGGCGCGAGCCCCTGATGCTCTTCGTCCAGATCATCCTGATC GACAAGACCGGCTTCCATCCGAGTACGTGCTCGCTCGATGCGATGTTTCGCTTGGT GGTCGAATGGGCAGGTAGCCGGATCAAGCGTATGCAGCCGCCGCATTGCATCAGCC ATGATGGATACTTTCTCGGCAGGAGCAAGGTGAGATGACAGGAGATCCTGCCCCGG CACTTCGCCCAATAGCAGCCAGTCCCTTCCCGCTTCAGTGACAACGTCGAGACAGC TGCGCAAGGAACGCCCGTCGTGGCCAGCCACGATAGCCGCGCTGCCTCGTCTTGG AGTTCATTCAGGGCACCGGACAGGTCGGTCTTGACAAAAAGAACCGGGCGCCCCT GCGCTGACAGCCGGAACACGGCGGCATCAGAGCAGCCGATTGTCTGTTGTGCCCA GTCATAGCCGAATAGCCTCTCCACCCAAGCGGCCGGAGAACCTGCGTGCAATCCAT CTTGTTCAATCATGCGAAACGATCCTCATCCTGTCTCTTGATCAGATCTTGATCCCCT GCGCCATCAGATCCTTGGCGGCAAGAAAGCCATCCAGTTTACTTTGCAGGGCTTCC CAACCTTACCAGAGGGCGCCCCAGCTGGCAATTCCG SEQ ID NO: 23 |

### Example 2. Preparation of strains having enhanced activity of aromatic amino acid transporter and confirmation of L-valine production capability

An experiment was conducted to enhance the aroP gene by additionally inserting the aroP gene into the chromosome of the valine-producing strain *Corynebacterium glutamicum* KCCM11201P (Patent No. US 8465962 B).

### Example 2-1. Preparation of strains with additional insertion of aroP gene

*Corynebacterium glutamicum* KCCM11201P was transformed with the pDC24_ΔNCgl0867up::Ppyk _aroP and pDC24_ΔNCgl0867up::Pald_aroP vectors prepared in Example 1-1 by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/l of kanamycin. Thereafter, the transformed *Corynebacterium glutamicum* strain in which secondary recombination had been completed, was subjected to PCR using the primers of SEQ ID NO: 15 and SEQ ID NO: 16, and the nucleotide sequence was verified to confirm whether the additional gene had been inserted. Strains from the parent strain KCCM11201P in which the Ppyk_aroP and Pald_aroP genes were additionally inserted into the chromosome were named *Corynebacterium glutamicum* KCCM11201P_ΔNCgl0867up::Ppyk_aroP and KCCM11201P_ΔNCgl0867up::Pald_aroP, respectively.

**[Table 4]**

| SEQ ID NO: | Name | Sequence |
|---|---|---|
| 15 | Primer | CGACATGGAACTAACTCTCCTAG |
| 16 | Primer | CACTTATGATCGCTGTGATCAGG |

### Example 2-2. Confirmation of L-valine production capability of strains with additional insertion of aroP gene

In order to compare the valine production capability of the valine-producing strain *Corynebacterium glutamicum* KCCM11201P and the two strains prepared in Example 2-1, KCCM11201P_ΔNCgl0867up::Ppyk_aroP and KCCM11201P_ΔNCgl0867up::Pald_aroP, a flask evaluation was performed. After subculturing each strain in nutrient medium, each strain was inoculated into a 250 ml corner-baffle flask containing 25 ml of production medium, and cultured at 30 °C for 72 hours with shaking at 200 rpm. The final OD, valine production yield, and relative sugar consumption rate of each strain were measured and are shown in Table 5 below.

### [Nutrient medium (pH 7.2)]

10 g glucose, 5 g meat extract, 10 g polypeptone, 2.5 g sodium chloride, 5 g yeast extract, 20 g agar, 2 g urea (per liter of distilled water)

### [Production medium (pH 7.0)]

100 g glucose, 40 g ammonium sulfate, 2.5 g soy protein, 5 g corn steep solids, 3 g urea, 1 g potassium phosphate dibasic, 0.5 g magnesium sulfate heptahydrate, 100 µg biotin, 1 mg thiamine-HCl, 2 mg calcium pantothenate, 3 mg nicotinamide, 30 g calcium carbonate (per liter of distilled water)

**[Table 5]**

| Strain | FN OD | Valine yield | Valine yield increase (compared to the parent strain) | Relative sugar consumption rate | Tyrosine concentration | Phenylalanine concentration |
|---|---|---|---|---|---|---|
| | 562 nm | % | % | % | mg/L | mg/L |
| KCCM11201P (Parent strain) | 71.8 | 2.7 | - | 100 | 70 | 190 |
| KCCM11201P_ΔNC gl0867up::Ppyk_aroP | 68.7 | 3.1 | 114.81 | 111 | 0 | 0 |
| KCCM11201P_ΔNC gl0867up::Pald_aroP | 70.5 | 2.8 | 103.7 | 105 | 30 | 10 |

As shown in Table 5, when the aroP gene was additionally inserted to enhance the expression of aroP, both strains were superior to KCCM11201P in terms of the relative sugar consumption rate, with no decrease in the yield, and it was confirmed that the aromatic amino acids phenylalanine and tyrosine were reduced. Therefore, it was possible to confirm the effect of improving valine production capability and reducing by-products through enhancement of aroP expression. In particular, when the aroP gene operably linked to the Ppyk promoter was additionally inserted, it was confirmed that not only the sugar consumption rate increased but also the valine yield was improved by 15%.

### Example 2-3. Evaluation of valine production capability of Corynebacterium glutamicum CJ7V strain

In order to confirm whether the effect of the enhancement of aroP also increases valine production capability in other valine-producing strains belonging to *Corynebacterium glutamicum*, a strain having improved valine production capability was prepared by introducing one type of mutation [ilvN(A42V); Biotechnology and Bioprocess Engineering, June 2014, Volume 19, Issue 3, pp 456-467] into the acetolactate synthase isozyme 1 small subunit (IlvN) protein of wild-type *Corynebacterium glutamicum* ATCC14067.

First, in order to prepare a vector for introducing the A42V mutation into the *ilvN* gene, genomic DNA of the wild-type *Corynebacterium glutamicum* ATCC14067 strain was extracted using a G-spin Total DNA extraction mini kit according to the manufacturer's protocol. PCR was performed using the genomic DNA as a template and using the primer pair of SEQ ID NO: 17 and SEQ ID NO: 18 and the primer pair of SEQ ID NO: 19 and SEQ ID NO: 20 to obtain gene fragments A and B, respectively. PCR conditions consisted of denaturation at 94 °C for 5 minutes; 28 repetitions of denaturation at 94 °C for 30 seconds, annealing at 55 °C for 30 seconds, and polymerization at 72 °C for 60 seconds; and then polymerization was performed at 72 °C for 7 minutes. As a result, a 528 bp gene fragment H and a 509 bp gene fragment I were obtained. Overlapping PCR was performed using the obtained gene fragments H and I as templates and the primer pair of SEQ ID NO: 17 and SEQ ID NO: 20. As a result, a 1010 bp PCR product (hereinafter named "mutation-introduced fragment J") was obtained.

The mutation-introduced fragment J obtained above was treated with restriction enzyme SmaI, ligated with the pDC24 vector treated with the same restriction enzyme, and transformed into *E. coli* DH5α by electroporation to induce homologous recombination on the chromosome. Strains in which the vector was inserted into the chromosome by recombination of homologous sequences were selected on LB medium containing kanamycin. DNA was obtained from the selected transformed *E. coli* strain using a DNA-spin plasmid DNA purification kit according to the manufacturer's protocol, and a pDC24-ilvN(A42V) vector for introducing the A42V mutation of the *ilvN* gene, comprising the mutation-introduced fragment J, was prepared.

The primer sequences used here are as shown in Table 6 below.

**[Table 6]**

| SEQ ID NO: | Sequence Name | Sequence (5' -> 3') |
|---|---|---|
| 17 | primer | cggggatcccccgggAGGACGGTACTCAAATACTAAACTTC |
| 18 | primer | TGCCGAGTGTTTCGGTCTTTACAGACACGAGGGACACG |
| 19 | primer | TGTCTGTAAAGACCGAAACACTCGGCATCAA |
| 20 | primer | cggggatcccccgggGACAACTACATTATTATTATACCACA |

Wild-type *Corynebacterium glutamicum* ATCC14067 was transformed with the pDC24-ilvN(A42V) vector prepared above by homologous recombination on the chromosome. Strains in which the vector was inserted into the chromosome by recombination of homologous sequences were selected on a medium containing 25 mg/l of kanamycin. Thereafter, PCR was performed using the primer pair of SEQ ID NO: 17 and SEQ ID NO: 20 on the transformed *Corynebacterium glutamicum* strain in which secondary recombination had been completed to amplify a gene fragment, and then a strain in which the A42V mutation had been introduced into the *ilvN* gene was confirmed through gene sequence analysis. The recombinant strain was named *Corynebacterium glutamicum* CJ7V.

Finally, the *Corynebacterium glutamicum* CJ7V was transformed with the pDC24-ΔNCgl0867up::Ppyk_aroP vector in the same manner as in Example 2-1. The recombinant strain was named *Corynebacterium glutamicum* CJ7V-ΔNCgl0867up::Ppyk_aroP.

The valine production capability of the parent strain CJ7V and the CJ7V-ΔNCgl0867up::Ppyk_aroP strain was evaluated in the same manner as in Example 2-2, and is shown in Table 7 below.

**[Table 7]**

| Strain | FN OD | Valine yield | Valine yield increase (compared to the parent strain) | Relative sugar consumption rate | Tyrosine concentration | Phenylalanine concentration |
|---|---|---|---|---|---|---|
| | 562 nm | % | % | % | mg/L | mg/L |
| CJ7V (Parent strain) | 137.3 | 2.2 | 100 | 100 | 80 | 160 |
| CJ7V-ΔNCgl0867u p::Ppyk_aroP | 131 | 2.5 | 113.64 | 109 | 0 | 0 |

As a result, it was reconfirmed that when aroP of the *Corynebacterium glutamicum* microorganism was enhanced, the relative sugar consumption rate increased, the production of by-products (tyrosine, phenylalanine) decreased, and the valine yield was increased.

From the above description, those skilled in the art to which the present disclosure pertains will be able to understand that the present disclosure can be embodied into different and more detailed modes, without departing from the technical spirit or essential features thereof. In this regard, the exemplary embodiments disclosed herein are only for illustrative purposes and should not be construed as limiting the scope of the present invention. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A gene expression cassette comprising:
(a) one or more promoters selected from the group consisting of a promoter of a pyruvate kinase (pyk) gene, a promoter of an acetaldehyde dehydrogenase (ald) gene, a promoter of a cytochrome c oxidase subunit 3 (ctaE) gene, and a promoter of an ATP-dependent 6-phosphofructokinase isozyme 1 (pfkA) gene; and
(b) a gene encoding an aromatic amino acid transporter operably linked to the promoter.

2. The gene expression cassette according to claim 1, wherein the promoter of the pyk gene comprises the nucleotide sequence of SEQ ID NO: 3, the promoter of the ald gene comprises the nucleotide sequence of SEQ ID NO: 4, the promoter of the ctaE gene comprises the nucleotide sequence of SEQ ID NO: 21, and the promoter of the pfkA gene comprises the nucleotide sequence of SEQ ID NO: 22.

3. The gene expression cassette according to claim 1, wherein the gene encoding the aromatic amino acid transporter is derived from a microorganism of the genus *Corynebacterium*.

4. A microorganism having enhanced activity of an aromatic amino acid transporter and increased valine production capability.

5. The microorganism according to claim 4, wherein the aromatic amino acid transporter is derived from a microorganism of the genus *Corynebacterium*.

6. The microorganism according to claim 4, wherein the enhancement is accomplished by (i) overexpression of a gene encoding the aromatic amino acid transporter, (ii) operable linkage of a gene encoding the aromatic amino acid transporter to a strong promoter, or (iii) a combination thereof.

7. The microorganism according to claim 6, wherein the overexpression of the gene is conducted by additionally introducing the gene into the microorganism.

8. The microorganism according to claim 6, wherein the strong promoter is one or more selected from the group consisting of a promoter of a pyk gene, a promoter of an ald gene, a promoter of a ctaE gene, and a promoter of a pfkA gene.

9. The microorganism according to claim 8, wherein the promoter of the pyk gene comprises the nucleotide sequence of SEQ ID NO: 3, the promoter of the ald gene comprises the nucleotide sequence of SEQ ID NO: 4, the promoter of the ctaE gene comprises the nucleotide sequence of SEQ ID NO: 21, and the promoter of the pfkA gene comprises the nucleotide sequence of SEQ ID NO: 22.

10. The microorganism according to claim 4, wherein the microorganism is a microorganism of the genus *Corynebacterium*.

11. The microorganism according to claim 10, wherein the microorganism of the genus *Corynebacterium* is *Corynebacterium glutamicum*.

12. The microorganism according to any one of claims 4 to 11, wherein the microorganism has increased valine production capability, compared to a parent strain or wild-type microorganism in which the activity of the aromatic amino acid transporter is not enhanced.

13. A method for producing valine, comprising culturing the microorganism according to any one of claims 4 to 11 in a medium.

14. The method for producing valine according to claim 13, further comprising recovering valine from the medium resulting from the culturing or the microorganism.

15. A method for increasing valine production, comprising culturing the microorganism according to any one of claims 4 to 11 in a medium.
